# EUROPEAN PATENT APPLICATION

(11) **EP 4 613 253 A1**
(43) Date of publication of application: **10.09.2025**
(21) Application number: 24161218.3
(22) Date of filing: 04.03.2024
(51) Int. Cl.: A61G 13/12, A61B 6/04

(54) **A RADIOLUCENT ATTACHMENT, A PATIENT SUPPORT APPARATUS, AND A RADIOLUCENT COUPLING PORTION**

(71) Applicant: Baxter Medical Systems GmbH + Co. KG, 07318 Saalfeld (DE)
(72) Inventor: KUCHENBECKER, Arnd, Saalfeld (DE); LIMBACH, Tobias, Saalfeld (DE); KOOIKER, Heather, Saalfeld (DE)
(74) Representative: Reddie & Grose LLP

(57) **Abstract**

A radiolucent attachment for a patient support apparatus, the attachment comprising: a supporting surface for supporting a portion of a patient, the supporting surface having a recess configured to removably receive one of a plurality of radiolucent inserts; and a radiolucent coupling portion, configured to cooperate with a corresponding radiolucent coupling portion of a patient support apparatus or a patient support apparatus section, for coupling the attachment to the patient support apparatus. A patient support apparatus and a radiolucent coupling portion for a radiolucent attachment are also provided.

## Description

### TECHNICAL FIELD

The present disclosure relates to a radiolucent attachment for a patient support apparatus. In particular, the disclosure relates to a radiolucent attachment having a radiolucent coupling portion, configured to cooperate with a corresponding radiolucent coupling portion of a patient support apparatus or a patient support apparatus section, and a supporting surface having a recess configured to removably receive one of a plurality of radiolucent inserts

### BACKGROUND

Person support apparatuses, such as surgical tables or operating room (OR) tables, hospital beds, and gurneys, are used throughout care settings.

Patients commonly have to undergo medical imaging, in particular before, during, and after surgery. Medical imaging is normally performed in a dedicated room having an imaging apparatus, such as a X-ray machine, a computed tomography (CT) apparatus, or a magnetic resonance imaging (MRI) apparatus. Increasingly, medical imaging is performed during surgery, in particular during robot-assisted surgery, or immediately before surgery, when the patient has already been positioned for surgery.

Specialised person support apparatuses are available for medical imaging, such as movable scanning tables which can be used to move the patient into a medical imaging apparatus. Such scanning tables are radiolucent, permitting electromagnetic radiation, and in particular X-rays, to pass through the scanning table.

However, moving a patient to a scanning table, in particular during surgery, may result in medical imaging in a position that is different to the patient's position during surgery. Surgical procedures may also result in movement of bodily tissue and fluids, which may affect the usefulness of medical imaging prior to surgical intervention.

While person support apparatuses such as surgical tables with radiolucent sections are known, such surgical tables are limited in their functionality and often allow medical imaging only of small portions of a patient's body. There is also limited compatibility of such person support apparatus with other components.

There is a particular clinical need for radiolucent head / upper body sections, i.e. sections configured to support a head / upper body of a patient. Such head / upper body sections are usually configured to be attached to back sections of surgical or OR tables. Because back sections of surgical or OR tables are typically radiopaque, their overall radiolucency is limited.

The inventors have appreciated the need for radiolucent attachments, such as radiolucent head / upper body sections, to allow for improved medical images to be produced of large portions of a patient's body. In particular, the inventors have appreciated the need for a radiolucent attachment which allows for improved medical images during upper body, neck, and head surgery.

The inventors have further appreciated the need for medical imaging during surgery with a patient, and patient's head, in various positions, including in prone position and supine position. This may allow a radiolucent attachment to be used for cosmetic surgery, head trauma, neck surgery, angiography, fluoroscopy, as well as for surgery of infants and babies.

### SUMMARY OF THE DISCLOSURE

The present disclosure provides a radiolucent attachment for a patient support apparatus, a patient support apparatus, and a radiolucent coupling portion for a radiolucent attachment. Optional features are defined in the appended dependent claims.

According to a first aspect of the present disclosure, there is provided a radiolucent attachment for a patient support apparatus. The attachment comprises: a supporting surface for supporting a portion of a patient, the supporting surface having a recess configured to removably receive one of a plurality of radiolucent inserts; and a radiolucent coupling portion, configured to cooperate with a corresponding radiolucent coupling portion of a patient support apparatus or a patient support apparatus section, for coupling the attachment to the patient support apparatus.

By providing a radiolucent attachment which comprises a radiolucent coupling portion configured to cooperate with a corresponding radiolucent coupling portion of the patient support apparatus or a patient support apparatus section, the radiolucent attachment according to the first aspect permits improved medical images of a large body part to be produced during surgery.

By providing a recess configured to removably receive one of a plurality of radiolucent inserts, the radiolucent attachment may allow for a patient to be positioned and imaged in a variety of surgical positions. In particular, depending on the surgical procedure, receiving a radiolucent insert selected from a variety of interchangeable radiolucent inserts, may allow for improved imaging during surgery in a greater variety of positions.

The recess may alternatively be referred to as a cut-out, in particular as a universal cut-out, configured to removably receive one of a plurality of radiolucent inserts, which may be referred to as interchangeable forms, or interchangeable add-ins.

The radiolucent attachment may be configured, and/or sized, to support a patient's head, neck, and/or shoulders. The radiolucent attachment may be a radiolucent head segment. The radiolucent head segment may be referred to as a radiolucent head section.

When the radiolucent attachment is a head segment/section, or in which the radiolucent attachment is configured to support a patient's head, neck, and/or shoulders, the recess may allow for different radiolucent inserts to be received, to enable different head positioning during medical imaging. As such, a head segment/section according to the disclosure may be more versatile than known head segments, and may provide for improved medical imaging in a variety of positions.

The radiolucent coupling portion may allow for the radiolucent attachment to be coupled to an existing radiolucent attachment, or radiolucent section, of a patient support apparatus. In particular, radiolucent pelvic attachments or radiolucent pelvic sections of patient support apparatuses are often available. The pelvic attachments or sections are usually configured to receive lower body/leg attachments - however, the radiolucent coupling portion of the present disclosure may permit for a head/upper body segment/section to be coupled to the pelvic attachment/section. This may improve medical imaging of an upper body and/or head of a patient, because the radiolucent head segment/section may be coupled to a radiolucent pelvic attachment/section, rather than to a, typically radiopaque, back section.

Optionally, the patient support apparatus may be an OR table or a surgical table.

The radiolucent attachment may further comprise a radiolucent insert coupling portion for engaging a corresponding radiolucent coupling portion of one of the plurality of radiolucent inserts, for coupling the insert to the attachment. Advantageously, by providing a radiolucent insert coupling portion, a modular attachment permitting for a variety of radiolucent inserts to be received may be provided. This may allow for improved medical imaging in a variety of positions.

Optionally, the insert coupling portion comprises a locking mechanism for securing the insert in the recess. Advantageously, a locking mechanism may allow for an insert to be secured relative to the recess, minimising movement during medical imaging, thus improving the quality of medical imaging. It is understood that the locking mechanism may allow for selective locking and unlocking.

The locking mechanism may comprise at least one of: a retractable or sprung projection configured to engage a corresponding aperture of the one of the plurality of radiolucent inserts; an aperture configured to be engaged by a corresponding retractable or sprung projection of the one of the plurality of radiolucent inserts; a tapered pin configured to engage a corresponding aperture of the one of the plurality of radiolucent inserts; a clamping mechanism configured to engage a corresponding clamping portion of the one of the plurality of radiolucent inserts; a clamping portion configured to be engaged by a corresponding clamping mechanism of the one of the plurality of radiolucent inserts; and a threaded aperture configured to be engaged by a threaded portion of a fastening member of the one of the plurality of radiolucent inserts.

The insert coupling portion may be configured to slidably receive the one of the plurality of radiolucent inserts. Advantageously, slidably receiving the radiolucent insert may facilitate inserting and removing of inserts from the insert coupling portion. It may also reduce the risk of incorrect installation.

Optionally, the insert coupling portion comprises at least one of: a groove configured to be engaged by a corresponding projecting element of the insert; and/or a projecting element configured to engage a corresponding groove of the insert. Advantageously, providing a groove on the insert coupling portion may reduce any risk of damage to the insert coupling portion when no insert is inserted into the attachment, i.e. when the recess is unoccupied. The groove and/or projecting element may be elongate.

The insert coupling portion may comprise a lip, or a flange. The lip, or the flange, may be configured so that a radiolucent insert can be placed into the recess from above. The lip, or the flange, may be configured to engage a corresponding recess, or lip, or flange, of the one of the plurality of radiolucent inserts.

The attachment may be formed of a radiolucent material which is non-metallic. Optionally, the radiolucent material is one of: a polymer; wood, in particular plywood; a laminated material, such as phenolic paper; and a fibre reinforced composite. Advantageously, wood based materials may be inexpensive and easily machined. Advantageously, fibre reinforced composites may be inexpensive and have low density but high strength.

When the radiolucent material is a fibre reinforced composite, the fibre reinforced composite may be a carbon fibre reinforced composite and/or a fibre reinforced polymer. Advantageously, a carbon fibre reinforced composite is a durable radiolucent material.

In one example, the radiolucent material may be a carbon fibre reinforced polymer, such as a carbon fibre reinforced thermoplastic.

Optionally, the radiolucent coupling portion comprises at least one radiolucent projection configured to be inserted into a corresponding radiolucent receiving portion of the patient support apparatus or the patient support apparatus section. Advantageously, the radiolucent coupling portion comprising at least one radiolucent projection may enable the patient support apparatus or the patient support apparatus section to not require a projection and merely require receiving portions, thus avoiding portions of the patient support apparatus or the patient support apparatus section projecting when no attachment is coupled, reducing risk of damage.

Additionally or alternatively, the radiolucent coupling portion comprises a radiolucent receiving portion configured to receive a corresponding radiolucent projection of the patient support apparatus or the patient support apparatus section.

The or each radiolucent projection may be tapered. Advantageously, providing a tapered radiolucent projection may facilitate insertion into the corresponding radiolucent receiving portion. In particular, the or each radiolucent projection may be tapered in a proximal direction.

As used herein, the term "distal" may refer to portions of the radiolucent attachment which are closer to the patient support apparatus when the radiolucent attachment is coupled to the patient support apparatus. As used herein, the term "proximal" may refer to portions of the radiolucent attachment which are further from the patient support apparatus when the radiolucent attachment is coupled to the patient support apparatus.

The or each radiolucent projection may have a rectangular cross-section. Advantageously, a rectangular cross-section may prevent or reduce tilting. Optionally, the rectangular cross-section may be a square cross-section.

Alternatively, the or each radiolucent projection may have a circular cross-section. Advantageously, a circular cross-section may be easily manufactured.

Optionally, the or each radiolucent projection comprises a securing means for releasably securing the respective projection in the corresponding receiving portion. Advantageously, a securing means may prevent the attachment from being inadvertently moved relative to the patient support apparatus.

Optionally, the securing means is formed of a different material than the projection. Further optionally, the securing means is formed of a polymer, in particular an injection moulded polymer and a remainder of the projection is formed of a carbon fibre reinforced polymer.

In one particular example, the securing means may comprise: a core comprising carbon fibre, in particular a carbon fibre web; and moulded epoxy.

Optionally, the securing means comprises a resilient member. The resilient member may be configured to be deflectable from a default, securing, position to a deflected, non-securing, position. The resilient member may be configured to prevent movement of the projection into and/or out of the corresponding receiving portion when the resilient member is in the default position. The resilient member may be biased into the default, securing, position.

The resilient member may be configured to permit for the projection to be moved into and/or out of the corresponding receiving portion when the resilient member is in the deflected position.

The resilient member may be configured so that moving the projection into the corresponding receiving portion deflects the resilient member into the deflected position. In particular, the resilient member, or a portion of the resilient member, may be shaped so that moving the projection into the corresponding receiving portion deflects the resilient member into the deflected position. The resilient member may be tapered.

The resilient member may comprise a spring. Advantageously, the spring may bias the resilient member into the default, securing position.

Alternatively or additionally, the resilient member may be configured to be inherently resilient. In particular, the resilient member may be made of a flexible material. That is, the resilient member may allow for the required deflection to the non-securing position and restoration to the default, securing, position without the need for further components, such as springs. The flexible material may comprise a polymer.

Optionally, the resilient member comprises a locking member configured to engage with a corresponding locking portion of the receiving portion. Advantageously, a locking member and corresponding locking portion may allow for selectively securable engagement of the projection and the receiving portion.

Optionally, the locking member comprises a locking protrusion, and the locking portion comprises a corresponding slot. Alternatively or additionally, the locking member comprises a latch or a hook, and the locking portion comprises a corresponding latch receiver or a corresponding hook receiver.

The radiolucent attachment may further comprise two radiolucent projections, provided at a first end of the attachment, at opposite lateral ends of the first end of the attachment. Advantageously, two radiolucent projections arranged as described may allow for secure attachment of the radiolucent attachment and prevents tilting using a minimum number of projections.

The radiolucent attachment may comprise at least one rail configured to receive a, optionally radiolucent, carriage of a peripheral device for slidably coupling the peripheral device to the radiolucent attachment. Advantageously, by allowing a peripheral device to be attached to the radiolucent attachment, versatility of the radiolucent attachment may be improved.

The carriage may comprise a C-shaped attaching portion for engaging the rail.

Optionally, the peripheral device comprises at least one of: a diagnostic device, such as an angiography device; and a display device, such as a vital sign display; and a head support, configured to support at least a head of a patient. Regarding the head support, it may be beneficial for the radiolucent attachment to be configured to receive a torso of a patient, while the head is supported separately. This may be particularly important if medical imaging of the torso is required. The head support may be radiolucent.

The at least one rail may be arranged laterally on the radiolucent attachment. Optionally, the radiolucent attachment comprises a first rail arranged at a first lateral end end and a second rail arranged on a second lateral end, opposite the first lateral end.

Optionally, the insert coupling portion comprises the at least one rail, and/or the first rail and the second rail.

Optionally, a, or the, insert coupling portion is provided adjacent the recess. The insert coupling portion may border the recess.

The recess may have a substantially triangular shape. The recess may have a curved shape, optionally a semi-circular shape.

The recess may have a substantially rectangular shape. Where the recess has a rectangular shape, the recess may be open on one side. Optionally, at least two closed sides comprise a portion of the insert coupling portion.

Optionally, at least one of the corners of the rectangular recess is curved. Curved corners may reduce force concentration around the corners. Advantageously, by providing curved corners, durability of the attachment may be improved.

Optionally, the supporting surface comprises a protruding support section configured to form a continuous surface when inserted into a corresponding cutout section of a supporting surface of the patient support apparatus or the patient support apparatus section.

Advantageously, by providing a continuous support surface, a patient on the patient support apparatus may be supported evenly.

Optionally, the radiolucent attachment further comprises a radiolucent insert configured to be received by the radiolucent attachment. In particular, the radiolucent attachment may comprise a radiolucent insert received in the recess of the radiolucent attachment.

The radiolucent insert may comprise the corresponding coupling portion for engaging the insert coupling portion of the attachment. In particular, the radiolucent attachment may comprise a radiolucent insert received in the recess of the radiolucent attachment, and coupled to the radiolucent attachment via the insert coupling portion and the corresponding coupling portion.

In some examples, the radiolucent insert may be a support plate, configured to combine with the supporting surface to form a continuous supporting surface. If the radiolucent insert is a support plate, the support plate optionally further comprises attaching means for attaching a radiolucent head section to the support plate.

In some examples, the radiolucent insert may be a head rest, comprising a band for bridging the open end of the recess. If the radiolucent insert is a head rest, the head rest optionally comprises a flat portion and a curved portion, the curved portion forming a channel for receiving a head, in particular a forehead, of the patient. Such a head rest, or resting bar or band, may allow a patient's face to remain unobstructed for easier breathing and access to the patient's airways.

In some examples, the radiolucent insert may comprise a circular portion for supporting a head of the patient.

In some examples, the radiolucent insert comprises a support plate having a circular recess, and the circular portion comprises at least one, radiolucent, ring reversibly insertable into the circular recess for supporting a head of the patient. By providing a circular recess, and at least one ring reversibly insertable into the circular recess, a patient's face may be accessible from beneath the attachment when the patient's head is supported, in a prone position, by the at least one ring. This may facilitate certain surgical interventions.

If the circular portion comprises more than one ring, the more than one rings may be stackable. In this way, a height at which the head of the patient is supported may be selectable.

The at least one ring, when inserted, may be configured to protrude from the support plate in a direction of a patient's head.

In some examples, the circular portion may comprise a ring-shaped protrusion. The circular portion may further comprise a dome, or cap, which may be substantially a spherical dome or cap, arranged opposite the ring-shaped protrusion. The spherical dome/cap and ring-shaped protrusion may be particularly suitable for supporting a patient's head when the patient is in a supine position.

According to a second aspect of the present disclosure, there is provided a patient support apparatus comprising a radiolucent attachment according to the first aspect.

The patient support apparatus may be a surgical table or a OR table.

Optionally, the patient support apparatus comprises a radiolucent support section to which the radiolucent attachment is coupled.

The radiolucent support section may be a radiolucent pelvic section/segment or a radiolucent torso section/segment.

The radiolucent support section optionally comprises a supporting surface having a, or the, corresponding cutout section, in particular if the radiolucent attachment comprises the protruding support section.

The radiolucent support section may comprise the corresponding radiolucent receiving portion, in particular if the radiolucent attachment comprises the at least one radiolucent projection.

The corresponding radiolucent receiving portion may comprise a corresponding locking portion of the receiving portion, in particular if the radiolucent attachment comprises the resilient member comprising the locking member.

The corresponding locking portion comprises the corresponding slot, in particular if the locking member comprises the locking protrusion.

According to a third aspect of the present disclosure, there is provided a radiolucent coupling portion for a radiolucent attachment according to the first aspect. The radiolucent coupling portion comprises: at least one radiolucent projection configured to be inserted into a corresponding radiolucent receiving portion, for coupling components comprising the projection and the corresponding receiving portion.

The, or each, projection may be tapered. Advantageously, providing a tapered radiolucent projection may facilitate insertion into the corresponding radiolucent receiving portion. In particular, the or each radiolucent projection may be tapered in a proximal direction.

The, or each, radiolucent projection may have a rectangular cross-section. Advantageously, a rectangular cross-section may prevent or reduce tilting. The rectangular cross-section may be a square cross-section.

Alternatively, the, or each radiolucent projection may have a circular cross-section. Advantageously, a circular cross-section may be easily manufactured.

The, or each, radiolucent projection, may comprise a securing means for releasably securing the respective projection in the corresponding receiving portion. Advantageously, a securing means may prevent the component comprising the radiolucent coupling portion from being inadvertently moved relative to the component comprising the corresponding radiolucent receiving portion.

Optionally, the projection is formed of a radiolucent material which is non-metallic. The radiolucent material may be one of: a polymer; wood, in particular plywood; a laminated material, such as phenolic paper; and a fibre reinforced composite. Advantageously, wood based materials may be inexpensive and easily machined. Advantageously, fibre reinforced composites may be inexpensive, low density, and have high strength.

When the radiolucent material is a fibre reinforced composite, the fibre reinforced composite may be a carbon fibre reinforced material and/or a fibre reinforced polymer. Optionally, the fibre reinforced composite may be a carbon fibre reinforced thermoplastic. Advantageously, carbon fibre reinforced thermoplastic is a durable radiolucent material.

Optionally, the securing means is formed of a different material than the remaining projection. Further optionally, the securing means is formed of a polymer, in particular an injection moulded polymer and a remainder of the projection is formed of a carbon fibre reinforced polymer.

The securing means may comprise: a core comprising carbon fibre, in particular a carbon fibre web; and moulded epoxy.

Optionally, the securing means comprise a resilient member. The resilient member may be configured to be deflectable from a default, securing, position to a deflected, non-securing, position. The resilient member may be configured to prevent movement of the projection into and/or out of the corresponding receiving portion when the resilient member is in the default position. The resilient member may be biased into the default, securing, position.

The resilient member may be configured to permit for the projection to be moved into and/or out of the corresponding receiving portion when the resilient member is in the deflected position.

The resilient member may be configured so that moving the projection into the corresponding receiving portion deflects the resilient member into the deflected position. In particular, the resilient member may be shaped so that moving the projection into the corresponding receiving portion deflects the resilient member into the deflected position. The resilient member may be tapered.

The resilient member may comprise a spring. Advantageously, the spring may bias the resilient member into the default, securing position.

Alternatively or additionally, the resilient member may be configured to be inherently resilient. In particular, the resilient member may be made of a flexible material. That is, the resilient member may allow for the required deflection to the non-securing position and restoration to the default, securing, position without the need for further components, such as springs. The flexible material may comprise a polymer.

Optionally, the resilient member comprises a locking member configured to engage with a corresponding locking portion of the receiving portion. Advantageously, a locking member and corresponding locking portion may allow for a selectively securable engagement of the projection and the receiving portion.

Optionally, the locking member comprises a locking protrusion, and the locking portion comprises a corresponding slot in the receiving portion. Alternatively or additionally, the locking member comprises a latch or a hook, and the locking portion comprises a corresponding latch receiver or a corresponding hook receiver.

The radiolucent attachment may comprise two radiolucent projections, provided at a first end of the attachment, at opposite lateral ends of the first end of the attachment. Advantageously, two radiolucent projections arranged as described may allow for secure attachment of the radiolucent attachment and prevents tilting using a minimum number of projections.

Further features of the third aspect of the present disclosure are described above in relation to the first aspect of the present disclosure. It will be understood by those skilled in the art that features described above with relation to the radiolucent coupling portion present in the first aspect of the present disclosure may be applied, *mutatis mutandis*, to the radiolucent coupling portion of the third aspect of the present disclosure.

According to a fourth aspect of the present disclosure, there is provided a radiolucent coupling comprising a radiolucent coupling portion according to the third aspect, and a corresponding radiolucent receiving portion.

The corresponding radiolucent receiving portion may be tapered. Optionally, the corresponding radiolucent receiving portion may have a rectangular, in particular a square, cross-section.

Further features of the fourth aspect of the present disclosure are described above in relation to the first and third aspects of the present disclosure. It will be understood by those skilled in the art that the features described above with relation to the radiolucent coupling portion present in the first aspect of the present disclosure may be applied, *mutatis mutandis*, to the corresponding radiolucent receiving portion present in the fourth aspect of the present disclosure.

### BRIEF DESCRIPTION OF DRAWINGS

The disclosure will be further described, by way of example only, with reference to the accompanying drawings, in which:
Figure 1 shows a perspective view of a patient support apparatus;
Figure 2 shows a perspective view of a portion of the patient support apparatus of Figure 1;
Figure 3 shows a perspective view of a patient support apparatus according to the present disclosure;
Figure 4 shows a perspective view of a radiolucent attachment according to the present disclosure, positioned for attachment to the patient support apparatus of Figure 2;
Figure 5 shows a perspective view of a radiolucent attachment according to the present disclosure;
Figures 6a and 6b show a detailed perspective side view and a perspective partly cut-away side view of a radiolucent coupling portion according to the present disclosure;
Figures 7a to 7c show detailed cross-sectional views of a radiolucent coupling according to the present disclosure;
Figures 8a shows a perspective view of a radiolucent attachment according to the present disclosure having a first radiolucent insert, and Figure 8b shows an enlarged cross-sectional view of the radiolucent attachment of Figure 8b;
Figure 9 shows a perspective view of a radiolucent attachment according to the present disclosure having a second radiolucent insert;
Figures 10a and 10b show perspective views of a radiolucent attachment according to the present disclosure having a third radiolucent insert; and
Figures 11a and 11b show perspective views of radiolucent attachments according to the present disclosure with a patient in supine and prone position, respectively.

### DETAILED DESCRIPTION OF DRAWINGS

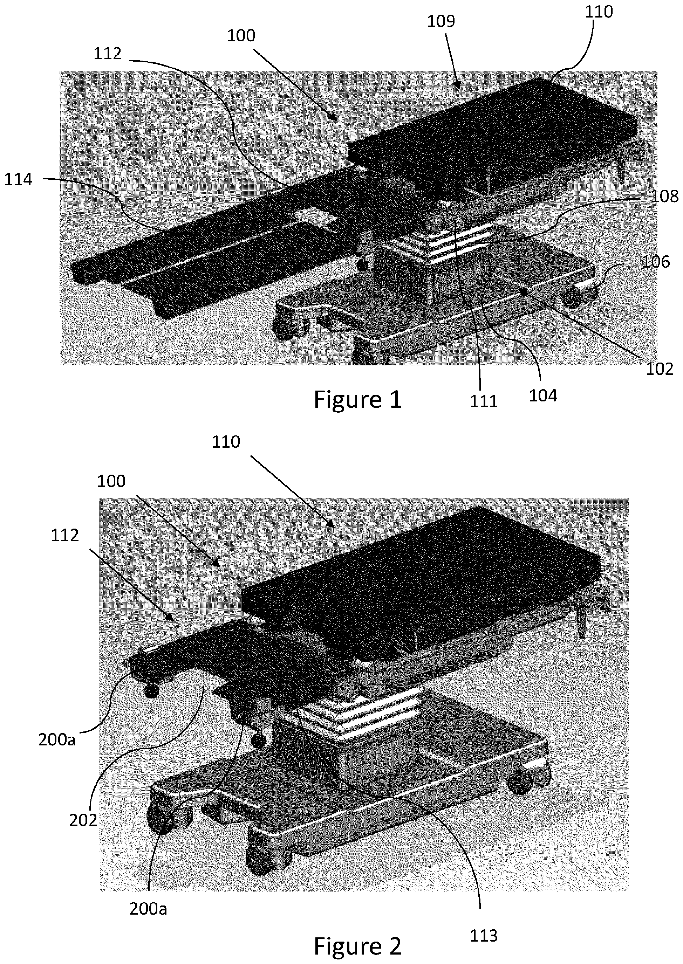

Figure 1 shows a patient support apparatus 100, specifically a surgical table. The patient support apparatus 100 comprises a main module 102 comprising a base 104. At each of its four corners, the base 104 comprises caster wheels 106 allowing for the patient support apparatus 100 to be moved. A column 108 couples a main module 109 of the patient support apparatus to the base 104. The main module 109 has a main supporting surface 110.

The main supporting surface 110 of the main module 109 is configured to support an upper body of a patient during a surgical procedure. Attached to the main module 109, by hook couplers 111, is a pelvic extension segment 112 having a pelvic support surface 113, the pelvic support surface 113 configured to support a pelvis of a patient during a surgical procedure. The pelvic extension segment 112 is radiolucent, permitting medical imaging to be carried out during surgery and/or with a patient supported on the patient support apparatus 100 in surgical position.

Attached to the pelvic extension segment 112 is a leg extension segment 114, the leg extension segment 114 configured to support legs of a patient being supported on the patient support apparatus 100 during a surgical procedure.

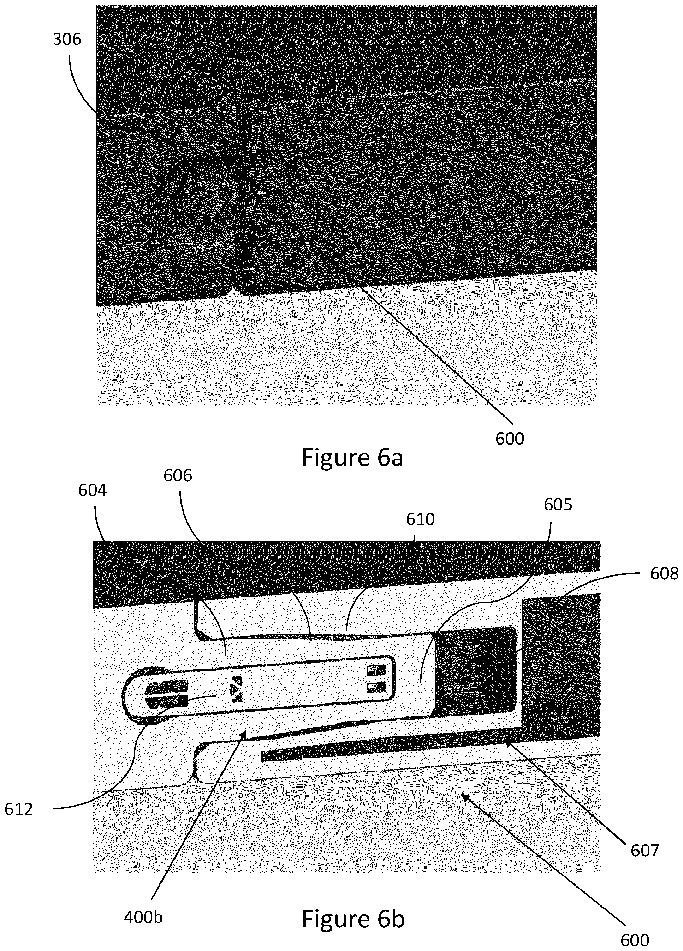

Figure 2 shows the patient support apparatus 100 with the leg extension segment removed. As can be more easily seen from Figure 2, the pelvic extension segment 112 comprises a pair of substantially square openings 200a, 200b, which form part of a radiolucent coupling 600, which will be discussed in detail below with reference to Figures 6a to 7c. The pelvic support surface 113 of the pelvic extension segment 112 comprises an isosceles-trapezoid cut-out 202.

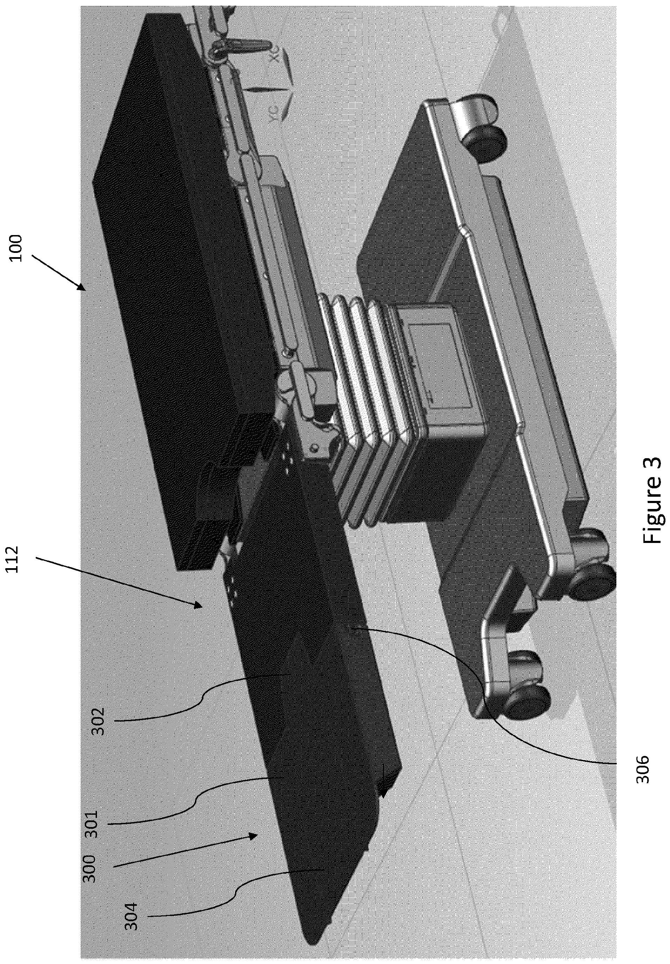

Figure 3 shows the patient support apparatus 100 having the pelvic extension segment 112. Attached to the pelvic extension segment 112 is a radiolucent attachment 300, particularly a modular radiolucent head / upper body section. The radiolucent attachment 300 has a support surface 301 configured to support a head and shoulder portion of a patient supported on the patient support apparatus 100. It will be appreciated that the radiolucent attachment 300 may be configured to, or may be used to, support another part of a body of a patient.

The radiolucent attachment 300 is made of a carbon fibre reinforced composite.

In the example shown in Figure 3, the radiolucent attachment 300 is configured to support a head and shoulder portion of a patient, with the pelvic extension segment 112 supporting a torso of the patient and the main supporting surface 110 supporting a pelvic and lower body portion of the patient.

The support surface 301 comprises a protruding support section 302, sized to fit into the isosceles-trapezoid cut-out 202. When the radiolucent attachment 300 is coupled to the pelvic extension segment 112, the pelvic support surface 113 and the support surface 301 of the radiolucent attachment 300 form a continuous support surface.

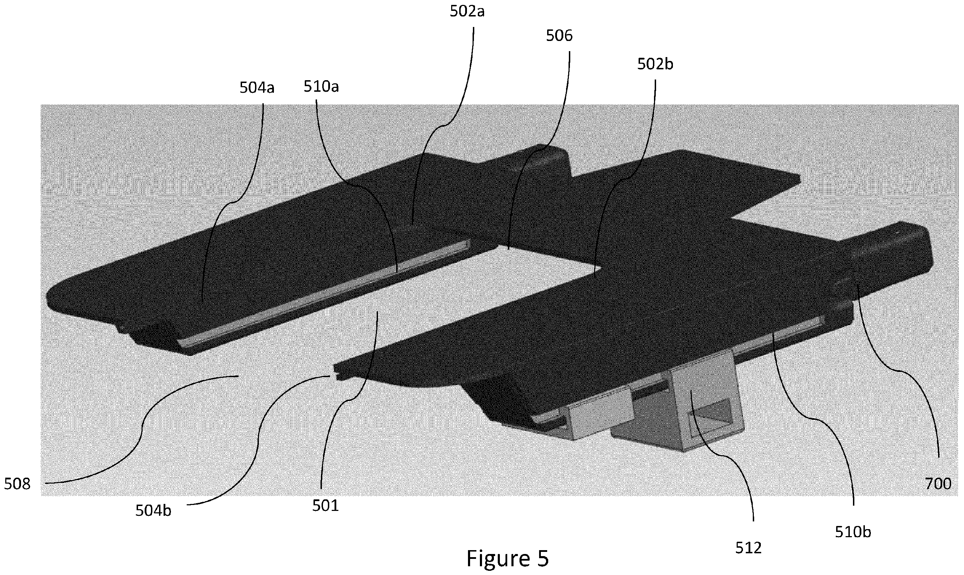

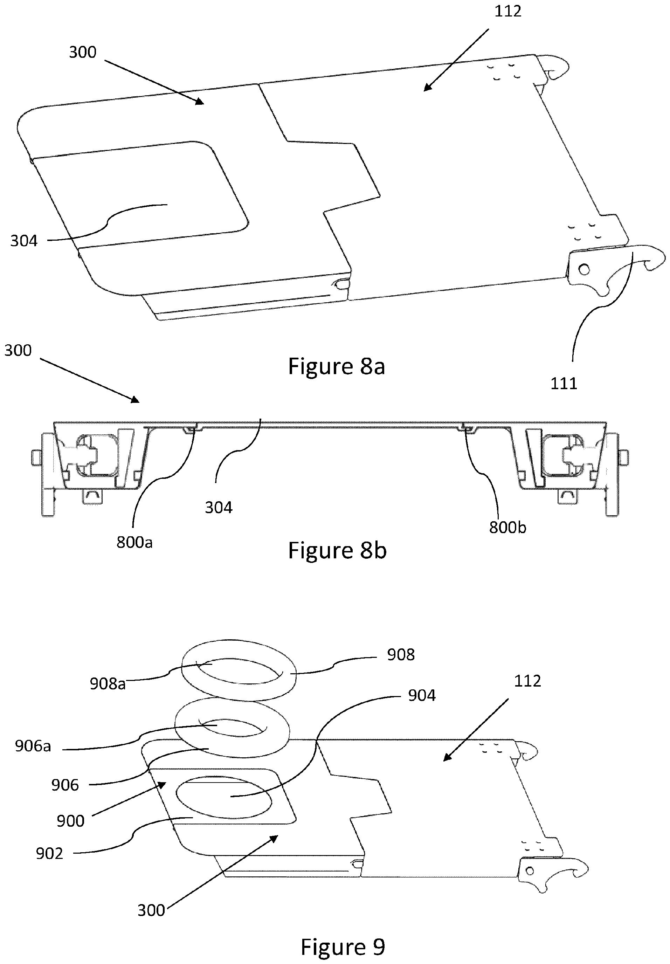

Inserted into a recess 501 in the support surface 301, as best seen in Figure 5, is a radiolucent insert 304. The radiolucent insert 304 is a support plate, configured to combine, when inserted into the recess 501, with the support surface 301 to form a continuous supporting surface. Different types of radiolucent inserts will be discussed below, with reference to Figures 8 to 11b.

The radiolucent coupling 600, which will be discussed in detail below, comprises a release button 306, configured to unlock the radiolucent coupling 600. When the release button 306 is pushed, the radiolucent attachment 300 may be removed from the pelvic extension segment 112, as will be discussed in more detail below.

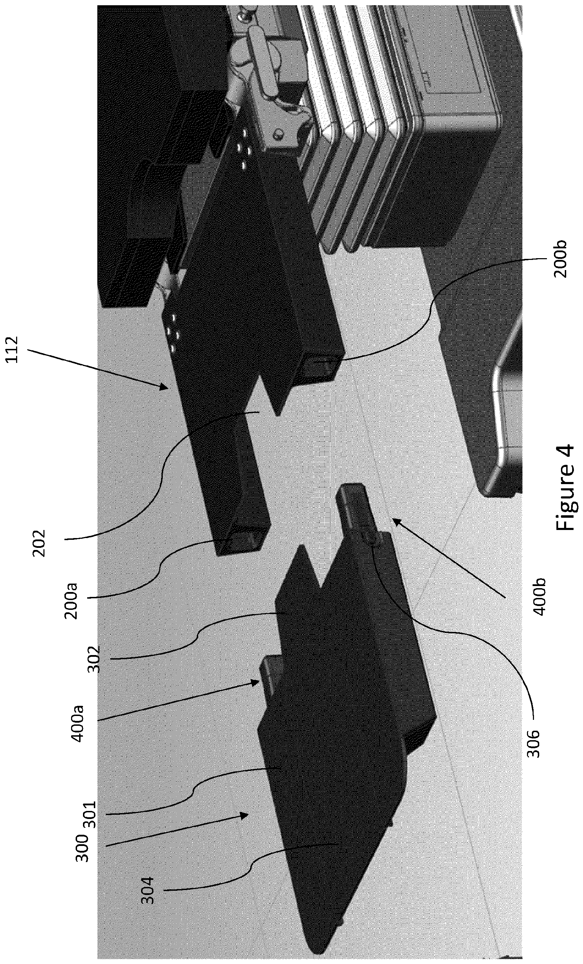

As shown in Figure 4, the radiolucent attachment 300 comprises two radiolucent projections 400a, 400b, arranged at a proximal end of the radiolucent attachment 300, at opposite lateral ends of the proximal end of the radiolucent attachment 300. Each of the radiolucent projections 400a, 400b comprises a respective release button 306 (only one visible in Figure 4).

Figure 5 shows a radiolucent attachment 500 which is substantially identical to radiolucent attachment 300. The radiolucent attachment 500 has a recess 501 configured to removably receive one of a plurality of radiolucent inserts. The recess 501 is substantially rectangular, with rounded corners 502a, 502b.

On each lateral boundary of the recess 501, i.e. those which are not the closed boundary 506 and the open boundary 508 of the recess 501, the radiolucent attachment 500 comprises a respective groove 504a, 504b, formed between a projecting portion of the support surface 301 and a second projecting portion. As best seen in Figure 8b, the radiolucent insert 304 comprises elongate projections 800a, 800b configured to engage the respective grooves 504a, 504b to slidably insert the radiolucent insert 304 into the recess 501. Although not shown in Figure 5, it is understood that the radiolucent insert 304 and/or the radiolucent attachment 500 may comprise a locking mechanism, for locking the radiolucent insert 304 in position.

The radiolucent attachment 500 further comprises a first rail 51 0a and a second rail 510b. The rails 510a, 510b are arranged at opposite lateral ends for the radiolucent attachment. The rail 510a slidably receives a carriage 512 having a C-shaped attaching portion. The carriage 512 may carry a peripheral device, such as a diagnostic device; a display device; or a head support, to be attached to the radiolucent attachment 500.

The carriage 512 may comprise a securing means for selectively securing the carriage 512 in position along the rail 510a.

A second carriage 514 is shown in Figure 5. The second carriage 514 may be a stop, limiting slidable movement of carriage 512 along the rail. In some examples, the second carriage 514 may also be a carriage of a peripheral device.

Referring now to Figures 6a and 6b, which show the radiolucent coupling 600 which couples the radiolucent attachment 300/500 to the pelvic extension element 112. The radiolucent coupling 600 comprises the radiolucent projection 400b, shown in Figure 6b, and radiolucent projection 400a. The features described below with reference to radiolucent projection 400b apply equally to radiolucent projection 400a.

Radiolucent projection 400b has a substantially square cross section, which reduced from a distal portion 604 having a greater cross sectional area to a proximal portion 605 having a smaller cross sectional area, along a tapered portion 606.

The radiolucent coupling 600 further comprises a corresponding receiving portion 607 of the pelvic extension element 112. The corresponding receiving portion 607 comprises a receptacle 608 sized to receive the projection 400b. The receptacle 608 has a substantially square cross section to match the substantially square cross section of radiolucent projection 400b. Thus, receptacle 608 also comprises a tapered portion 610 to conform to the tapered portion 606 of the radiolucent projection 400b.

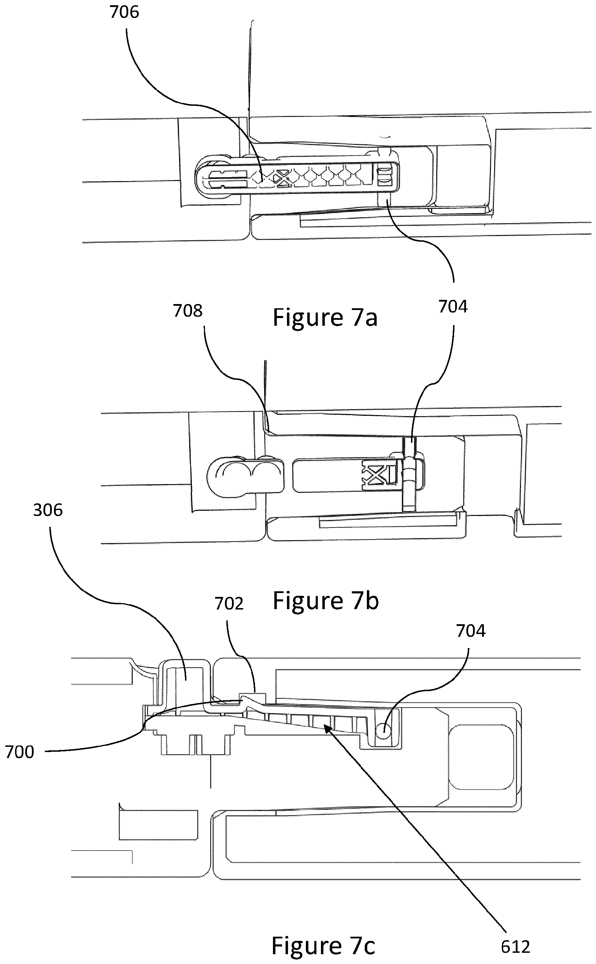

As best seen in Figure 6b, and further discussed below with reference to Figures 7a to 7c, the radiolucent projection 400b comprises a resilient member 612.

The resilient member 612, as best seen in Figure 7c, comprises a locking projection 700, also visible in Figures 4 and 5. The locking projection 700 is provided on the tapered portion 606 of radiolucent projection 400b. The locking projection 700 is configured to engage a corresponding locking slot 702 of the receptacle 608 of the corresponding receiving portion 607.

At a proximal end, the resilient member 612 is hingedly coupled to a hinge pin 704, permitting the resilient member 612 to be deflected inwardly, that is, towards a centre of the projection 400b (downwardly in Figure 7c). The resilient member 612 comprises button 306, at a distal end, for allowing the resilient member 612 to be easily deflected.

When deflected, the locking projection 700 is deflected along with the resilient member 612, thus permitting the radiolucent projection 400b to be moved into, or out of, the receptacle 608. The resilient member 612 is biased into the locking, or default, position.

The resilient member 612 is made of a different material than the remaining projection 400b. In particular, as best seen in Figure 7a, the resilient member 612 comprises a core 704 of carbon fibre web moulded with epoxy.

A mouth, that is the open, distal, end of the receptacle 608 is flared. The flared mouth 708 may facilitate insertion of the radiolucent projection 400b into the receptacle 608. Additionally or alternatively, the flared mouth 708 may allow for the resilient member 612 to be automatically deflected as the locking projection 700 is inserted into the receptacle 608.

As is also apparent from Figure 7c, the locking projection 700 has a tapered profile, that is a height of the locking projection 700 increases progressively from a proximal end of the locking projection 700 to a distal end of the projection 700. Thus, as the radiolucent projection 400b is pushed into the receptacle, the shape of the locking projection 700 may result in automatic deflection of the resilient member 612.

The pelvic extension segment 112 and the radiolucent attachment 300 shown in Figures 8a and 8b have already been described above.

Figure 9 shows the same radiolucent attachment 300, but in this example the radiolucent insert 304 has been replaced with a different radiolucent insert 900. This radiolucent insert 900 comprises a support surface 902 having a circular cut-out 904. The cut-out 904 may receive a patient's head, or may receive at least one radiolucent ring-shaped attachment of differing size 906, 908 for supporting a patient's head. The ring-shaped attachments 906, 908, when inserted into, or onto, the circular cut-out 904, are configured to project, in a direction of a patient's head, from the circular cut-out 904, to ensure that the patient's head is supported by the ring(s). As the ring-shaped attachments 906, 908 are of different size, a suitable ring may be selected depending on the patient and surgical needs.

The radiolucent ring-shaped attachments 906, 908 may be stackable, so that a position of the head 1100 of the patient 1102 may be further controlled by stacking at least two ring-shaped attachments 906, 908.

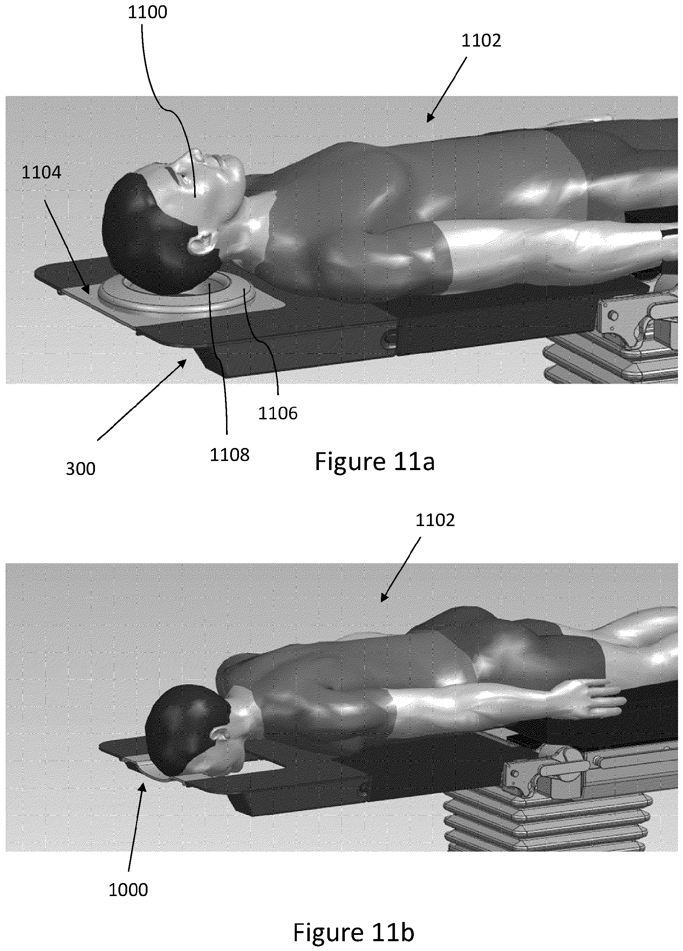

Figure 11a shows a head 1100 of a patient 1102 positioned on a similar radiolucent insert 1104 to radiolucent insert 900. As shown, radiolucent insert 900 with the at least one ring-shaped attachment 906, 908, and radiolucent insert 1104, may be particularly suitable for receiving a patient's head 1100 when the patient 1102 is in a supine position. However, radiolucent insert 900 may also be particularly suitable for receiving a patient's head 1100 when the patient 1102 is in a prone position, as the patient's face may be accessible through the respective cavity 906a, 908a in the respective ring(s) 906, 908.

It is noted that the radiolucent insert 1104 differs from radiolucent insert 900 in that the ring-shaped projection 1106 is fixed to, or uniformly formed with, the remaining portions of the radiolucent insert 1104, unlike the removably receivable rings 906, 908 of insert 900. The radiolucent insert 1104 also includes a part spherical cap 1108, configured to support a patient's head. It is noted that the ring-shaped projection 1106 and part spherical cap 1108 may cooperate to support a patient's head. Thus, insert 1104 may be particularly suitable for supporting a patient's head.

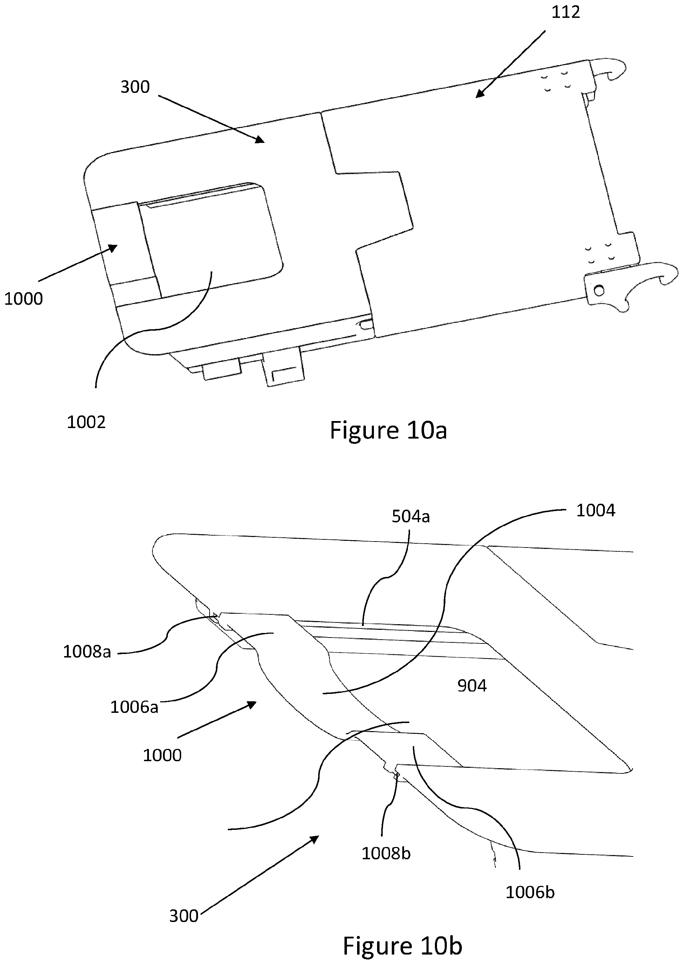

Figures 10a and 10b show the same radiolucent attachment 300, but in this example the radiolucent insert 304 or the radiolucent insert 900 has been replaced with a different radiolucent insert 1000. This radiolucent insert 1000 comprises a band for bridging the open boundary 508 of the recess 501. Thus, even with the radiolucent insert 1000 attached, the radiolucent attachment comprises a remaining recess 1002.

The radiolucent insert 1000 comprises a curved portion 1004 forming a channel for receiving a head of a patient, and flat portions 1006a, 1006b between which the curved portion 1004 is arranged.

As best seen from Figure 10b, at each end, the radiolucent insert 1000 comprises elongate projections 1008a, 1008b, which correspond to the elongate projections 800a, 800b of radiolucent insert 304.

As shown in Figure 11b, the radiolucent insert 1000 may be particular suitable for supporting a patient 1102 in a prone position, as the patient's face and airways are unobstructed.

The radiolucent insert 1000 may comprise a locking mechanism to secure the radiolucent insert 1000 relative to the respective grooves 504a, 504b. In some examples, the locking mechanism may comprise a fastener, such as a clamp or a screw. In some examples, the locking mechanism may comprise a stop, e.g. a thickened portion or a projection, at a distal end of at least one of the elongate projections 1008a, 1008b. The stop may prevent the distal end of the elongate projections 1008a, 1008b from entering the grooves 504a, 504b.

In use, the radiolucent attachment 300 is coupled to the radiolucent pelvic extension segment 112 by pushing the two radiolucent projections 400a, 400b into the respective corresponding receptacles 608 of the pelvic extension segment 112. As the projections 400a, 400b are pushed into the receptacles 608, the respective resilient members 612 are deflected to permit the projections 400a, 400b to advance into the receptacles 608 sufficiently so that the biased resilient members 612 restores to the default, locking, position as the locking projection 700 securely engages the corresponding locking slot 702.

Either before or after the radiolucent attachment 300 is coupled to the radiolucent pelvic extension segment 112, one of the radiolucent inserts 304, 900, 1000 may be inserted into the recess 501. Indeed, the radiolucent insert 304, 900, 1000 inserted into the recess 501 may be exchanged while the radiolucent attachment 300 is coupled to the radiolucent pelvic extension segment 112.

It will be apparent to those skilled in the art that while it has been described that the radiolucent attachment 300 is coupled to the pelvic extension segment 112 comprising the corresponding receiving portion 607, a corresponding receiving portion may be provided on any other suitable component of a patient support apparatus, such as a main module 109, or a torso extension segment.

Similarly, it will be apparent to those skilled in the art that while the radiolucent attachment 300 has been described as comprising the radiolucent projections 400a, 400b, the radiolucent attachment 300 may comprise more, or fewer, projections, or the radiolucent attachment 300 may comprise at least one of the receptacles 608 and the pelvic extension segment 112 may comprise at least one of the corresponding projections.

While the examples described above relate to adult patients, the radiolucent attachment according to the disclosure may be suitable for, or specifically configured for, paediatric surgery, in particular neonatal surgery.

It will be appreciated that the above described examples are exemplary of the disclosure only. Features described above in relation to one example of the disclosure may also be applied to other examples of the disclosure.

## Claims

1. A radiolucent attachment for a patient support apparatus, the attachment comprising:
a supporting surface for supporting a portion of a patient, the supporting surface having a recess configured to removably receive one of a plurality of radiolucent inserts; and
a radiolucent coupling portion, configured to cooperate with a corresponding radiolucent coupling portion of a patient support apparatus or a patient support apparatus section, for coupling the attachment to the patient support apparatus.

2. A radiolucent attachment according to claim 1, further comprising a radiolucent insert coupling portion for engaging a corresponding radiolucent coupling portion of one of the plurality of radiolucent inserts, for coupling the insert to the attachment, optionally wherein the insert coupling portion comprises a locking mechanism for securing the insert in the recess.

3. A radiolucent attachment according to claim 2, wherein the insert coupling portion is configured to slidably receive the one of the plurality of radiolucent inserts, and optionally wherein the insert coupling portion comprises at least one of:
a recess configured to be engaged by a corresponding projecting element of the insert; and/or
a projecting element configured to engage a corresponding recess of the insert.

4. A radiolucent attachment according to any preceding claim, wherein the attachment is formed of a radiolucent material which is non-metallic, the radiolucent material being one of:
a polymer;
wood, in particular plywood;
a laminated material, such as phenolic paper; and
a fibre reinforced composite, optionally a carbon fibre reinforced material and/or a fibre reinforced polymer, optionally a carbon fibre reinforced thermoplastic material.

5. A radiolucent attachment according to any preceding claim, wherein the radiolucent coupling portion comprises at least one radiolucent projection configured to be inserted into a corresponding radiolucent receiving portion of the patient support apparatus or the patient support apparatus section.

6. A radiolucent attachment according to claim 5, wherein the or each radiolucent projection is tapered and/or wherein the or each radiolucent projection has a rectangular, optionally square, cross-section.

7. A radiolucent attachment according to claim 5 or 6, wherein the or each radiolucent projection comprises a securing means for releasably securing the respective projection in the corresponding receiving portion, optionally wherein the securing means is formed of a different material than the projection, further optionally wherein the securing means is formed of a polymer, in particular an injection moulded polymer, and a remainder of the projection is formed of a carbon fibre reinforced polymer.

8. A radiolucent attachment according to claim 7, wherein the securing means comprise a resilient member, optionally wherein the resilient member:
is configured so that moving the projection into the corresponding receiving portion deflects the resilient member into a deflected position; and/or
comprises a locking member configured to engage with a corresponding locking portion of the receiving portion, optionally wherein the locking member comprises a locking protrusion.

9. A radiolucent attachment according to any of claims 5 to 8, comprising two radiolucent projections, provided at a first end of the attachment, at opposite lateral ends of the first end of the attachment.

10. A radiolucent attachment according to any preceding claim, comprising at least one groove configured to receive a, optionally radiolucent, carriage of a peripheral device for slidably coupling the peripheral device to the radiolucent attachment, optionally the peripheral device comprising at least one of:
a diagnostic device, such as an angiography device;
a display device, such as a vital sign display; and
a head support, configured to support at least a head of a patient.

11. A radiolucent attachment according to any preceding claim, wherein:
a, or the, insert coupling portion is provided adjacent the recess; and/or
the supporting surface comprises a protruding support section configured to form a continuous surface when inserted into a corresponding cutout section of a supporting surface of the patient support apparatus or the patient support apparatus section.

12. A radiolucent attachment according to any preceding claim, further comprising a radiolucent insert configured to be received by the radiolucent attachment, optionally the radiolucent insert comprising the corresponding coupling portion for engaging the insert coupling portion of the attachment.

13. A radiolucent attachment according to claim 12, wherein:
the radiolucent insert is a support plate, configured to combine with the supporting surface to form a continuous supporting surface; or
the radiolucent insert is a head rest, comprising a band for bridging the open end of the recess, optionally the head rest comprises a flat portion and a curved portion, the curved portion forming a channel for receiving a head of the patient; or
the radiolucent insert comprises a substantially circular portion for supporting a head of the patient, optionally wherein:
the radiolucent insert further comprises a support plate having a circular recess, and the circular portion comprises at least one, radiolucent, ring reversibly insertable into the circular recess for supporting a head of the patient, further optionally the more than one rings are stackable; or
the circular portion comprises a ring-shaped protrusion, further optionally wherein the circular portion further comprise a spherical cap, arranged opposite the ring-shaped protrusion.

14. A patient support apparatus comprising:
a radiolucent attachment according to any preceding claim; and optionally a radiolucent support section to which the radiolucent attachment is coupled, optionally wherein the radiolucent support section is a radiolucent pelvic section or a radiolucent torso section, further optionally wherein the radiolucent pelvic section or the radiolucent torso section comprises a supporting surface having the corresponding cutout section.

15. A radiolucent coupling portion for a radiolucent attachment according to any of claims 1 to 13, the radiolucent coupling portion comprising:
at least one radiolucent projection configured to be inserted into a corresponding radiolucent receiving portion, for coupling components comprising the projection and the corresponding receiving portion, wherein:
the, or each, projection is tapered, and the, or each, corresponding receiving portion is tapered; and/or
the, or each, radiolucent projection, and the, or each, corresponding receiving portion, has a rectangular, optionally square, cross-section; and/or
the, or each radiolucent projection, comprises a securing means for releasably securing the respective projection in the corresponding receiving portion, optionally wherein the securing means is a sprung portion.
